# FASCICULE DE BREVET EUROPEEN

(11) **EP 1 233 957 B1**
(45) Date de publication et mention de la délivrance du brevet: **22.02.2006**
(21) Numéro de dépôt: 00979732.5
(22) Date de dépôt: 15.11.2000
(51) Int. Cl.: C07D 333/38, A61K 31/381, A61P 25/00, A61P 25/28, A61P 25/16

(54) **DERIVES D'AMIDINES, LEUR PREPARATION ET LEUR APPLICATION A TITRE DE MEDICAMENTS**
AMIDIN-DERIVATE, IHRE HERSTELLUNG UND VERWENDUNG ALS MEDIKAMENTEN
NOVEL AMIDINE DERIVATIVES, PREPARATION AND USE THEREOF AS MEDICINES

(30) Priorité: 16.11.1999 FR 9914334
(43) Date de publication de la demande: 28.08.2002
(73) Titulaire: SOCIETE DE CONSEILS DE RECHERCHES ET D'APPLICATIONS SCIENTIFIQUES S.A. (S.C.R.A.S.), 75016 Paris (FR)
(72) Inventeur: CHABRIER DE LASSAUNIERE, Pierre-Etienne, 75016 Paris (FR); HARNETT, Jeremiah, 91190 Gif-sur-Yvette (FR)
(74) Mandataire: Bourgouin, André
(86) Numéro de dépôt international: PCT/FR2000/003168
(87) Numéro de publication internationale: WO 2001/036407

(56) Documents cités:
- WO-A-95/05363
- WO-A-96/18607
- WO-A-96/18608
- WO-A-97/46515
- WO-A-98/42696

## Description

La présente invention concerne de nouveaux dérivés d'amidines, leur préparation et leur application à titre de médicaments. Elle concerne en particulier l'utilisation desdits dérivés pour préparer un médicament destiné à inhiber les NO synthases (NOS) et/ou les monoamine oxydases (MAO).

Compte tenu du rôle potentiel des NOS et des MAO en physiopathologie, les nouveaux dérivés décrits répondant à la formule générale **(I)** peuvent produire des effets bénéfiques ou favorables dans le traitement de pathologies où ces deux enzymes sont impliquées. Sont concernées notamment les pathologies suivantes :
- les troubles du système nerveux central ou périphérique comme par exemple les maladies neurologiques où l'on peut notamment citer la maladie de Parkinson, les traumatismes cérébraux ou de la moelle épinière, l'infarctus cérébral, l'hémorragie sub arachnoïde, l'épilepsie, le vieillissement, les démences séniles, la maladie d'Alzheimer, la chorée de Huntington, la sclérose latérale amyotrophique, les neuropathies périphériques, la douleur ;
- la schizophrénie, les dépressions, les psychoses ;
- les troubles de la mémoire et de l'humeur ;
- les pathologies comme par exemple la migraine ;
- les troubles du comportement, la boulimie et l'anorexie ;
- les maladies auto-immunes et virales comme par exemple le lupus, le sida, les infections parasitaires et virales, le diabète et ses complications (notamment l'impuissance liée au diabète), la sclérose en plaques ;
- l'addiction aux substances toxiques ;
- les pathologies inflammatoires et prolifératives ;
- et plus généralement toutes les pathologies caractérisées par une production excessive des NOS et/ou une participation des MAO.

Dans l'ensemble de ces pathologies, il existe des évidences expérimentales démontrant l'implication des NOS (*J. Med. Chem.* (1995) **38**, 4343-4362) ainsi que l'implication des MAO (Goodman & Gilman's : *The pharmacological basis of therapeutics* , 9th ed., 1995,431-519).

Les inventeurs avaient déjà décrit dans des brevets antérieurs des inhibiteurs de NO Synthases et leur utilisation (brevet US 5,081,148 ; brevet US 5,360,925). La demande de brevet PCT WO 95/05363 décrit certains dérivés d'amidines et leur utilisation comme inhibiteurs de NO synthases. La demanderesse a elle-même décrit plus récemment d'autres dérivés d'amidines, lesquels inhibent les NO synthases et/ou piègent les formes réactives de l'oxygène (ROS pour *Reactive Oxygen Species*) (cf. notamment les demandes de brevet PCT WO 98/42696, WO 98/58934, WO 00/02860, WO 00/17190 et WO 00/17191).

La demanderesse vient à présent de découvrir que, de façon surprenante, les dérivés d'amidines répondant à la formule générale **(I)** définie ci-après sont des inhibiteurs des NOS et/ou des MAO.

Les composés de l'invention répondent à la formule générale **(I)** 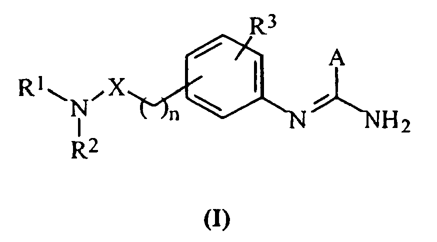
dans laquelle :
R¹ et R² représentent, indépendamment, un atome d'hydrogène ou un radical alkyle, cycloalkyle, alkényle, alkynyle, allènyle, allènylalkyle, cyanoalkyle, -(CH₂)_{g}-Z¹R⁴ ou -(CH₂)ₖ-COR⁵,
Z¹ représentant -O-, -NR⁶- , -S- ou une liaison,
R⁴ et R⁶ représentant, indépendamment, un atome d'hydrogène ou un radical alkyle, alkényle, allènylalkyle, alkynyle, alkoxy ou cyanoalkyle,
R⁵ représentant un radical alkyle, allènyle, allènylalkyle, alkényle, alkynyle, cyanoalkyle, alkoxy ou NR⁷R⁸,
R⁷ et R⁸ représentant, indépendamment, un atome d'hydrogène ou un radical alkyle, allènyle, allènylalkyle, alkényle, alkynyle, cyanoalkyle ou alkoxy,
ou R¹ et R² pris ensemble forment avec l'atome d'azote un hétérocycle non aromatique de 4 à 8 chaînons, les éléments de la chaîne étant choisis parmi un groupe composé de -CH(R⁹)-, -NR¹⁰-, -O-, -S-, -CO-, ledit hétérocycle pouvant être substitué par un ou plusieurs substituants -(CH₂)ₖ-Z²R¹¹ ou -(CH₂)ₖ-COR¹², ledit hétérocycle pouvant être par exemple une azétidine, une pipérazine, une homopipérazine, une 3,5-dioxopipérazine, une pipéridine, une pyrrolidine, une morpholine ou une thiomorpholine,
Z² représentant -O-, -NR¹³- ou -S- ou une liaison,
R¹¹, à chaque fois qu'il intervient, représentant indépendamment un atome d'hydrogène, un radical alkyle, alkényle, alkynyle, alkoxy, allènyle, allènylalkyle ou cyanoalkyle,
R¹³, à chaque fois qu'il intervient, représentant, indépendamment, un atome d'hydrogène ou un radical alkyle, alkényle, alkynyle, alkoxy, allènyle, allènylalkyle ou cyanoalkyle,
R¹², à chaque fois qu'il intervient, représentant un radical alkyle, allènyle, allènylalkyle, alkényle, alkynyle, cyanoalkyle, ou alkoxy ou NR¹⁴R¹⁵,
R¹⁴ et R¹⁵, à chaque fois qu'ils interviennent, représentant, indépendamment, un atome d'hydrogène ou un radical alkyle, alkoxy, allènyle, allènylalkyle, alkényle, alkynyle ou cyanoalkyle,
R⁹ et R¹⁰, à chaque fois qu'ils interviennent, représentant indépendamment un atome d'hydrogène, -(CH₂)ₖ-Z³R¹⁶ ou -(CH₂)ₖCOR¹⁷,
Z³ représentant -O-, -NR¹⁸-, -S- ou une liaison,
R¹⁸ représentant, inépendamment, un atome d'hydrogène ou un radical alkyle, alkényle, allènyle, allènylalkyle, alkynyle, alkoxy ou cyanoalkyle,
R¹⁶, à chaque fois qu'il intervient, représentant indépendamment un atome d'hydrogène ou un radical alkyle, alkényle, alkynyle, alkoxy, allènyle, allènylalkyle ou cyanoalkyle,
R¹⁷, à chaque fois qu'il intervient, représentant indépendamment un radical alkyle, allènyle, allènylalkyle, alkényle, alkynyle, cyanoalkyle, alkoxy ou NR¹⁹R²⁰,
R¹⁹ et R²⁰ représentant, indépendamment, à chaque fois qu'il interviennent, un atome d'hydrogène ou un radical alkyle alkoxy, allènyle, allènylalkyle, alkényle, alkynyle ou cyanoalkyle ;
X représente un radical -CO- ou -(CH₂)ₘ- ;
R³ représente un atome d'hydrogène ou un radical alkyle ou alkoxy linéaire ou ramifié ayant de 1 à 6 atomes de carbone ;
A représente un radical alkyle linéaire ou ramifié ayant de 1 à 6 atomes de carbone ou un radical aryle carbocyclique ou hétérocyclique à 5 ou 6 chaînons contenant de 1 à 4 hétéroatomcs choisis parmi O, S et N, et notamment les radicaux thiophène, furanne, pyrrole ou thiazole, ledit radical aryle étant éventuellement substitué par un ou plusieurs groupes choisis parmi les radicaux alkyle, alkényle ou alkoxy linéaires ou ramifiés ayant de 1 à 6 atomes de carbone ;
g, à chaque fois qu'il intervient, représentant, indépendamment, un entier de 1 à 6, m, k et n, à chaque fois qu'ils interviennent, représentant, indépendamment, des entiers de 0 à 6 ;
étant entendu toutefois que, lorsque R³ représente un atome d'hydrogène ou un radical alkyle linéaire ou ramifié ayant de 1 à 6 atomes de carbone, alors R¹ et R² ne représentent pas, indépendamment, un atome d'hydrogène ou un radical alkyle, et NR¹R² ne représente en outre pas l'un des groupes pipéridinyle, morpholinyle, pyrrolidinyle non substitués ou le groupe pipérazinyle optionnellement substitué en position 4 par un radical alkyle comptant de 1 à 6 atomes de carbone.

Par alkyle, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par allènyle, on entend le radical -CH=C=CH₂. Par aryle carbocyclique ou hétérocyclique, on entend un système carbocyclique ou hétérocyclique comprenant au moins un cycle aromatique, un système étant dit hétérocyclique lorsque l'un au moins des cycles qui le composent comporte un hétéroatome (O, N ou S). Par hétérocycle, on entend un système mono- ou polycyclique ledit système comprenant au moins un hétéroatome choisi parmi O, N et S et étant saturé, partiellement ou totalement insaturé ou aromatique. Par haloalkyle, on entend un radical alkyle dont au moins l'un des atomes d'hydrogène (et éventuellement tous) est remplacé par un atome halogène.

Par radicaux alkylthio, alkoxy, haloalkyle, haloalkoxy, aminoalkyle, alkényle, alkynyle, cyanoalkyle et aralkyle, on entend respectivement les radicaux alkylthio, alkoxy, haloalkyle, haloalkoxy, aminoalkyle, alkényle, alkynyle, cyanoalkyle et aralkyle dont le radical alkyle a la signification indiquée précédemment.

Par hétérocycle, on entend notamment les radicaux thiophène, pyrrole, pyrrolidine, furanne, tétrahydrofuranne, pipéridine, pipérazine, quinoline, indoline et indole. Par alkyle linéaire ou ramifié ayant de 1 à 6 atomes de carbone, on entend en particulier les radicaux méthyle, éthyle, propyle, isopropyle, butyle, isobutyle, *sec*-butyle et *tert*-butyle, pentyle, néopentyle, isopentyle, hexyle, isohexyle. Enfin, par halogène, on entend les atomes de fluor, de chlore, de brome ou d'iode.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

L'invention concerne en particulier les produits de formule générale **(I)** définie précédemment, dans lesquels se retrouvent, indépendamment, au moins l'une des caractéristiques suivantes :
- R¹ représentant un radical alkyle et R² représentant l'un des radicaux alkyle, cycloalkyle, alkényle, alkynyle, allènyle, allènylalkyle, cyanoalkyle, -(CH₂)_{g}-Z¹R⁴
ou -(CH₂)ₖ-COR⁵ tels que définis ci-dessus ;
- R¹ et R² pris ensemble formant avec l'atome d'azote un hétérocycle non aromatique de 4 à 8 chaînons, les éléments de la chaîne étant choisis parmi un groupe composé de -CH(R⁹)-, -NR¹⁰- et -O-,
   R⁹ et R¹⁰ représentant indépendamment un atome d'hydrogène ou un radical-(CH₂)ₖ-Z³R¹⁶ ou -(CH₂)ₖCOR¹⁷,
   Z³ représentant une liaison,
   R¹⁶ représentant indépendamment un atome d'hydrogène, un radical alkyle, alkényle, alkynyle, allènyle, allènylalkyle ou cyanoalkyle,
   R¹⁷ représentant indépendamment un radical alkyle, allènyle, allènylalkyle, alkényle, alkynyle ou cyanoalkyle ;
- n représentant 0 ou 1 ;
- k représentant un entier de 1 à 6 ;
- X représentant -(CH₂)ₘ- avec m représentant 0 ou 1.

De façon plus préférentielle, les produits de formule générale **(I)** définie précédemment, seront tels que se retrouvent, indépendamment, au moins l'une des caractéristiques suivantes :
- R¹ représentant un radical alkyle et R² représentant l'un des radicaux alkyle, alkényle, alkynyle, allènyle, allènylalkyle ou cyanoalkyle ;
- R¹ et R² pris ensemble forment avec l'atome d'azote un hétérocycle non aromatique de 4 à 8 chaînons, les éléments de la chaîne étant choisis parmi un groupe composé de -CH(R⁹)- et -NR¹⁰-,
   R⁹ et R¹⁰ représentant indépendamment un atome d'hydrogène ou un radical-(CH₂)ₖ-Z³R¹⁶,
   Z³ représentant une liaison,
   R¹⁶ représentant indépendamment un atome d'hydrogène ou un radical alkyle, alkynyle ou cyanoalkyle ;
- n représentant 0 ou 1 ;
- k représentant un entier de 1 à 3 ;
- X représentant -(CH₂)ₘ- avec m représentant 0 ou 1.

Des produits de formule générale **(I)** particulièrement préférés sont :
- le N'-(4-{[méthyl-(2-propynyl)amino]méthyl}phényl)-2-thiophènecarboximidamide ;
- le N'-(4-{[méthyl(cyanométhyl)amino]méthyl}phényl)-2-thiophènecarboximidamide ;
- le N'-(4-{[méthyl(propyl)amino]méthyl}phényl)-2-thiophènecarboximidamide ;
- le N'-(4-{[méthyl(3-cyanoéthyl)amino]méthyl}phényl)-2-thiophènecarboximidamide ;
- le N'-(4-{[méthyl(4-pentynyl)amino]méthyl}phényl)-2-thiophènecarboximidamide ;

et les sels de ces derniers.

Enfin, on préférera tout particulièrement le N'-(4-{[méthyl-(2-propynyl)amino]méthyl}phényl)-2-thiophènecarboximidamide ou les sels de ce dernier.

L'invention concerne également, à titre de médicaments, les composés de formule générale **(I)** ou leurs sels pharmaceutiquement acceptables. Elle a de plus pour objet les compositions pharmaceutiques contenant, à titre de principe actif, un composé de formule générale **(I)** ou un sel pharmaceutiquement acceptable d'un composé de formule générale **(I),** ainsi que l'utilisation des composés de formule générale **(I)** pour préparer un médicament destiné à inhiber les NO synthases et/ou les monoamine oxydases, en particulier la monoamine oxydase B.

En particulier, les composés de formule générale **(I)** peuvent être utilisés pour préparer un médicament destiné à traiter l'un des désordres ou l'une des maladies suivantes : la maladie de Parkinson, les démences séniles, la maladie d'Alzheimer, la chorée de Huntington, la sclérose latérale amyotrophique, la schizophrénie, les dépressions, les psychoses.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm.* (1986), **33**, 201-217.

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par le procédé décrit ci-après.

### PREPARATION DES COMPOSES DE L'INVENTION :

Les composés de formule générale **(I)** peuvent être préparés à partir des intermédiaires de formule générale **(II)** selon le schéma 1 où A, X, R¹, R², R³ et n sont tels que définis ci-dessus et Gp est un groupe protecteur de type carbamate. 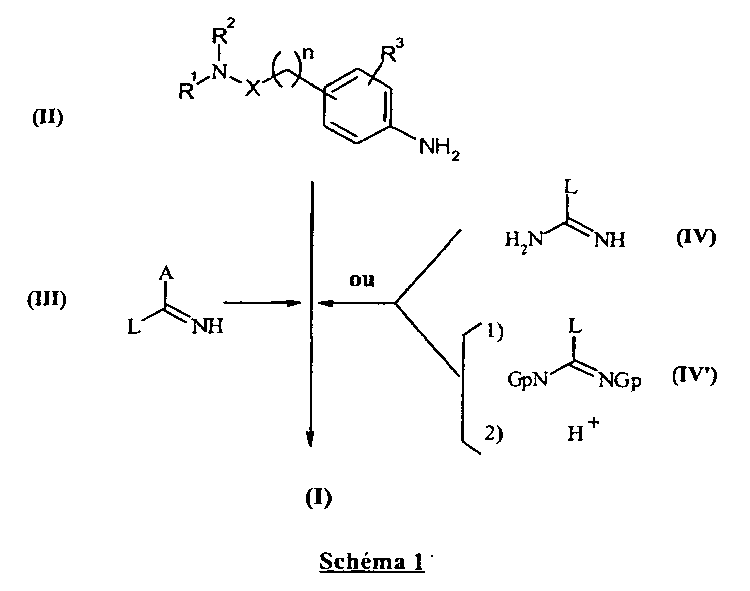
Les dérivés d'anilines de formule générale **(II),** peuvent être condensés sur des composés de formule générale **(III),** dans lesquels L représente un groupe partant (un radical alkoxy, alkylthio, acide sulfonique, halogénure, alcool arylique ou tosyle), pour conduire aux composés finaux de formule générale **(I)** de type amidine substitué (cf. schéma 1). Par exemple, pour A = thiophène, on peut condenser les dérivés de formule générale **(II)** sur l'iodhydrate de S-méthylthiophène thiocarboxamide, préparé selon une méthode de la littérature (*Ann. Chim.* (1962), **7**, 303-337). La condensation peut s'effectuer par chauffage dans un alcool (par exemple dans du méthanol ou de l'isopropanol), éventuellement en présence de DMF à une température de préférence comprise entre 50 et 100°C pour une durée généralement comprise entre quelques heures et une nuit.

Dans les cas où A est une amine, les composés finaux de formule générale **(I)** sont des guanidines. Celles-ci peuvent être préparées, par exemple, par la condensation des amines de formule générale **(II)** avec les dérivés de formule générale **(IV)** ou **(IV').** Les réactifs de formule générale **(IV),** dans lesquels L représente, par exemple, un cycle pyrazole, sont condensés sur les anilines de formule générale **(II)** selon les conditions décrites dans la littérature (*J. Org. Chem.* (1992) **57,** 2497-2502). On opère de même pour les réactifs de formule générale **(IV')** dans lesquels L représente, par exemple, un cycle pyrazole et Gp le groupement tBuOCO (*Tetrahedron Lett.* (1993) **34** (21), 3389-3392) ou bien lorsque L représente le groupement -N-SO₂-CF₃ et Gp le groupement tBuOCO (*J. Org. Chem.* (1998) **63,** 3804-3805). Lors de l'ultime étape de la synthèse, la déprotection de la fonction guanidine est effectuée en présence d'un acide fort tel que par exemple l'acide trifluoroacétique.

### Préparation des composés de formule générale (II) :

Les intermédiaires de formule générale **(II),** sont obtenus, par exemple, à partir de la réduction d'un précurseur de type nitro, comme illustré dans le schéma synthétique 2 ci-dessous.

### Réduction des précurseurs de type nitro :

La réduction de la fonction nitro des intermédiaires de formule générale **(V),** schéma 2, dans lesquels R¹, R², R³, X et n et sont tels que définis ci-dessus, est généralement effectuée par hydrogénation catalytique, dans l'éthanol, en présence de Pd/C, sauf dans les cas de molécules sensibles à ces conditions où le groupement nitro est sélectivement réduit, par exemple, en chauffant le produit dans un solvant approprié tel que l'acétate d'éthyle avec un peu d'éthanol en présence de SnCl₂ (*J. Heterocyclic Chem.* (1987), **24**, 927-930 ; *Tetrahedron Letters* (1984), **25** (8), 839-842) en présence de SnCl₂ / Zn (*Synthesis.* (1996),9,1076-1078) ou bien à l'aide de NaBH₄-BiCl₃ (*Synth. Com.* (1995) **25** (23), 3799-3803) dans un solvant tel que l'éthanol, ou alors en utilisant du Ni Raney additionné d'hydrate d'hydrazine (*Monatshefte für Chemie,* (1995), **126**, 725-732).

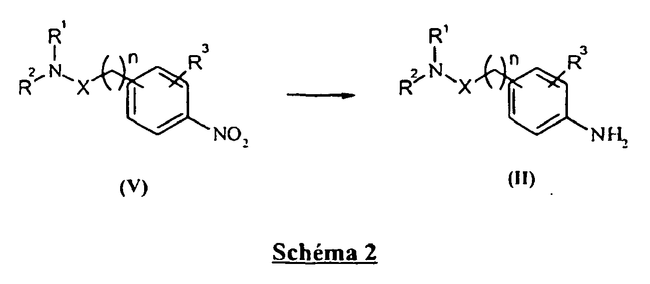

### Préparation des composés de formule générale (V) :

### Synthèses des carboxamides:

Les carboxamides de formule générale **(V),** schéma 3, dans lesquels R¹, R², R³, m et n sont tels que définis ci-dessus, sont préparés par condensation des acides de formule générale **(VI)** avec les amines de formule générale **(VII)** (schéma 3) ou des acides de formule générale **(VIII)** avec des amines de formule générale **(IX)** (schéma 3*bis*) dans les conditions classiques de la synthèse peptidique (M. Bodanszky et A. Bodanszky, The Practice of Peptide Synthesis, 145 (Springer-Verlag, 1984)), en particulier dans le THF, le dichlorométhane ou le DMF en présence d'un réactif de couplage tel que le dicyclohexylcarbodiimide (DCC), le 1,1'-carbonyldiimidazole (CDI) (*J. Med. Chem.* (1992), **35** (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)).

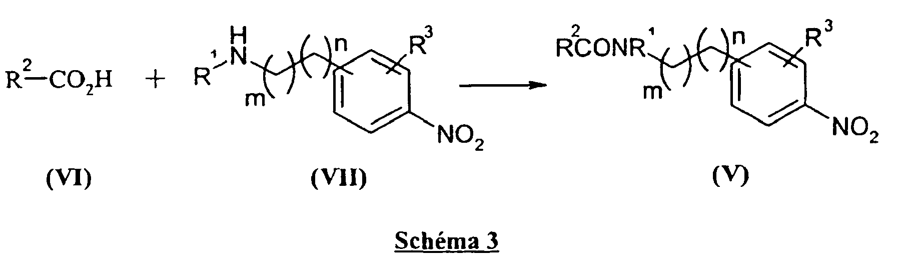

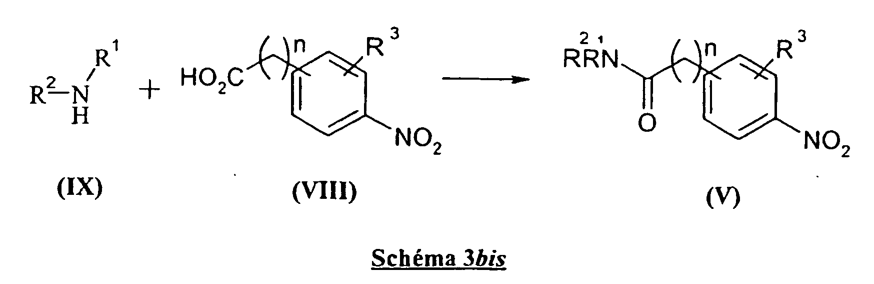

### Synthèse des amines de formule generale (V) :

Les amines de formule générale **(V),** schéma 4, dans lesquelles R¹, R², R³, m et n tels que définis ci-dessus, peuvent être préparées en une seule étape par condensation des amines de formule générale **(VII)** sur des dérivés halogénés de formule générale (X) (Hal représente un atome halogène) en présence d'une base telle que, par exemple, K₂CO₃ et/ou la triéthylamine, dans un solvant tel que, par exemple, l'acétonitrile.

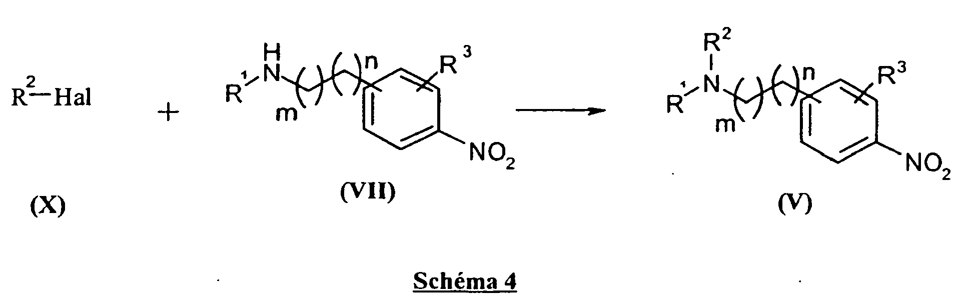

Lorsque R¹, R², R³, m et n sont tels que définis ci-dessus, les amines de formule générale **(V)** peuvent également être préparées à partir des dérivés halogénés de formule **(XI)** (Hal représente un atome halogène) et des amines de formule générale **(IX),** schéma 5, en présence d'une base telle que, par exemple, K₂CO₃ et/ou la triéthylamine, dans un solvant tel que, par exemple, l'acétonitrile.

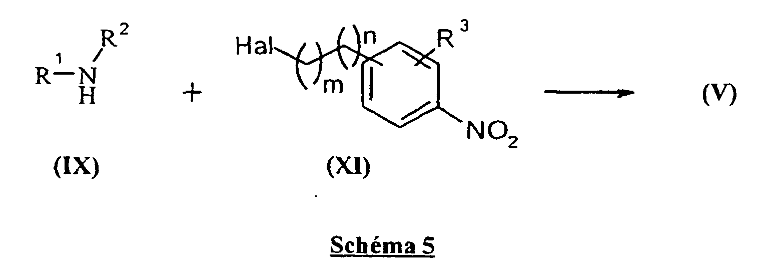

### Synthèse des amines par amination réductrice :

Les amines de formule générale **(V),** schéma 6, dans lesquelles R¹, R², R³, m et n sont tels que définis ci-dessus, peuvent être préparées par condensation d'un aldéhyde de formule générale **(XII)** avec une amine de formule générale **(VII)** en milieu réducteur. La réaction a lieu dans un solvant alcoolique tel que, par exemple, le méthanol en présence de tamis moléculaire 4 Å pulvérulent, préalablement activé, et d'un agent réducteur tel que, par exemple, NaBH₄ ou NaBH₃CN.

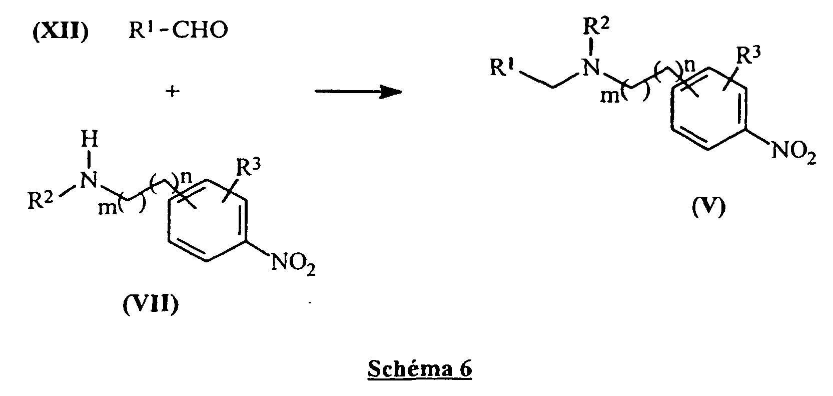

Les amines de formule générale **(V),** schéma 7, dans lesquelles R¹, R², R³ et n sont tels que définis ci-dessus, peuvent également être préparées par condensation d'une amine de formule générale **(IX)** avec un aldéhyde de formule générale **(XIII)** en milieu réducteur dans les conditions précédemment décrites.

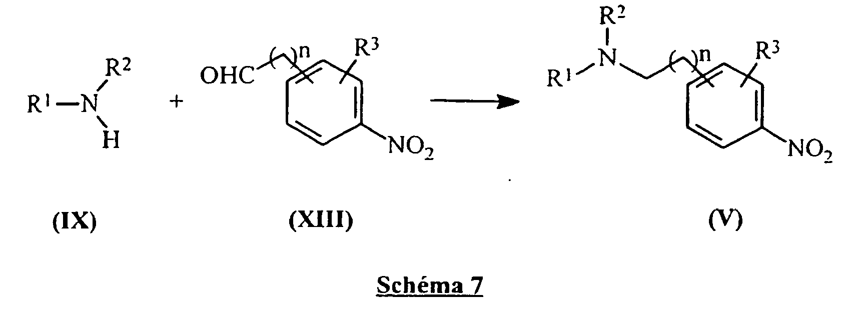

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

### Exemple 1 :N'-(4-{[méthyl(2-propynyl)amino]méthyl}phényl)-2-thiophènecarboximidamide

### 1.1. N-méthyl-N-(4-nitrobenzyl)-2-propyn-1-amine

A une solution de 1 g (1,45 mmol) de N-méthylpropargylamine dans 15 ml de dichlorométhane et 3,1 ml de triéthylamine, on ajoute, goutte-à-goutte, à 0°C, 3,72 g (22,0 mmol) de chlorure de p-nitrobenzyle dans 20 ml de dichlorométhane. Après une nuit d'agitation, le mélange réactionnel est concentré à sec sous vide, et le résidu est dilué avec du dichlorométhane et 40 ml d'une solution saturée de NaCl. Après agitation et décantation, la phase organique est séparée et séchée sur sulfate de magnésium, filtrée et concentrée sous vide. Le produit attendu est obtenu après chromatographie sur une colonne de silice, éluant : (20% acétate d'éthyle dans heptane), les fractions pures, après évaporation, donnent un solide marron avec un rendement de 80%. Point de fusion : 47-49 °C.
MH+ = 205,20.

### 1.2. 4-{[méthyl(2-propynyl)amino]méthyl}aniline

A une solution de 0,5 g (2,442 mmol) de l'intermédiaire 1.1 dans un mélange de 12,0 ml d'acide acétique glacial et 1,5 ml de HCl (12 N), on ajoute successivement 1,4 g (6.11 mmoles) de SnCl₂, 2H₂O et 0,4 g (6,11 mmol) de Zn. L'ensemble est agité 18 heures à 20 °C. Le mélange réactionnel est ensuite rendu basique par addition d'une solution aqueuse de NaOH à 30%. Le produit est alors extrait à l'aide de 2 fois 50 ml de CH₂Cl₂. La solution organique est lavée par 50 ml de saumure, séchée sur MgSO₄, filtrée et concentrée sous vide. On obtient une huile jaune pâle avec un rendement de 90%. Le résidu est utilisé sans autre purification dans l'étape suivante.
MH+ = 175,10.

### 1.3. N'-(4-{[méthyl(2-propynyl)amino]méthyl}phényl)-2-thiophènecarboximidamide

Dans un ballon de 25 ml, on dissout 0,315 g (1,80 mmol) de l'intermédiaire 1.2 et 0,77 g (2,70 mmol) d'iodhydrate de S-méthyl-2-thiophènethiocarboximide dans 15 ml d'isopropanol. Le mélange réactionnel est agité pendant 20 heures à une température de 80 °C. Après évaporation du solvant sous vide, le résidu est repris dans 25 ml d'un mélange (1/1) d'une solution saturée de NaHCO₃ et de dichlorométhane. Après décantation, la phase organique est lavée avec 2 x 25 ml de saumure. La solution organique est séchée sur sulfate de magnésium, filtrée, concentrée sous vide et le résidu est purifié sur une colonne de gel de silice (éluant : dichlorométhane + 3% d'éthanol). Les fractions pures sont collectées et concentrées sous vide. On obtient un solide blanc avec un rendement de 38%. Point de fusion : 109-110 °C.
MH+ = 284,10.

*Les composés suivants peuvent être préparés selon un protocole analogue à celui décrit dans l'exemple 1.*

### Exemple 2 : N'-(4-{[méthyl(cyanométhyl)amino]méthyl}phényl)-2-thiophènecarboximidamide

(la N-méthylpropargylamine est remplacée par le méthylaminoacétonitrile dans la première étape)

### Exemple 3: N'-(4-{[méthyl(propyl)amino]méthyl}phényl)-2-thiophènecarboximidamide

(la N-méthylpropargylamine est remplacée par la N-méthyl-N-propylaminc dans la première étape)

### Exemple 4 : N'-(4-{[methyl(3-cyanoéthyl)amino]methyl}phenyl)-2-thiophènecarboximidamide

(la N-méthylpropargylamine est remplacée par le N-méthyl-β-alaninenitrile dans la première étape)

### Exemple 5 : N'-(4-{[méthyl(4-pentynyl)amino]méthyl}phényl)-2-thiophènecarboximidamide

(la N-méthylpropargylamine est remplacée dans la première étape par la N-méthyl-N-pent-4-ynylamine (préparée selon *Tetrahedron Letters* (1992), **33**(45), 6835-6838, et *Heterocycles* (1996), **42**(1), 385-396))

### Etude pharmacologique des produits de l'invention

### Etude des effets sur la NO synthase constitutive neuronale de cervelet de rat

L'activité inhibitrice des produits de l'invention est déterminée par la mesure de leur effets sur la transformation par la NO synthase de la [³H]L-arginine en [³H]L-citrulline en accord avec la méthode modifiée de Bredt et Snyder (*Proc. Natl. Acad. Sci. USA,* (1990), **87**: 682-685). Des cervelets de rats Sprague-Dawley (300g - Charles River) sont rapidement prélevés, disséqués à 4 °C et homogénéisés dans un volume de tampon d'extraction (HEPES 50 mM, EDTA 1 mM, pH 7,4, pepstatine A 10 mg/ml, leupeptine 10 mg/ml). Les homogénats sont ensuite centrifugés à 21000 g pendant 15 min à 4 °C. Le dosage se fait dans des tubes à essai en verre dans lesquels sont distribués 100 µl de tampon d'incubation contenant 100 mM d'HEPES (pH 7,4), 2 mM d'EDTA, 2.5 mM de CaCl₂, 2 mM de dithiotréitol, 2 mM de NADPH réduit et 10 µg/ml de calmoduline. On ajoute 25 µl d'une solution contenant 100 nM de [³H]L-arginine (activité spécifique : 56,4 Ci/mmole, Amersham) et 40 µM de L-arginine non radioactive. La réaction est initiée en ajoutant 50 µl d'homogénat, le volume final étant de 200 µl (les 25 µl manquants sont soit de l'eau, soit le produit testé. Après 15 min, la réaction est stoppée avec 2 ml de tampon d'arrêt (20 mM d'HEPES ; pH 5,5 ; 2 mM d'EDTA). Après passage des échantillons sur une colonne de 1 ml de résine DOWEX, la radioactivité est quantifiée par un spectromètre à scintillation liquide. Le composé de l'exemple 1 décrit ci-dessus présente une Cl₅₀ inférieure à 3,5 µM.

### Etude des effets sur la liaison d'un ligand spécifique de la MAO-B, le [³H]Ro 19-6327

L'activité inhibitrice des produits de l'invention est déterminée par la mesure de leurs effets sur la liaison d'un ligand spécifique de la MAO-B, le [³H]Ro 19-6327.

### a) Préparation mitochondriale de cortex de rats

La préparation mitochondriale de cortex de rats est réalisée selon la méthode décrite dans Cesura A M, Galva M D, Imhof R et Da Prada M, *J Neurochem.* **48** (1987), 170-176. Les rats sont décapités et leurs cortex prélevés, homogénéisés dans 9 volumes d'un tampon sucrose 0,32 M tamponné à pH 7,4 avec 5 mM d'HEPES, puis centrifugés à 800 g pendant 20 minutes. Les surnageants sont récupérés et les culots lavés 2 fois avec le tampon sucrose 0,32 M comme précédemment. Les surnageants récoltés sont centrifugés à 10000g pendant 20 minutes. Les culots obtenus sont mis en suspension dans un tampon Tris (50 mM Tris, 130 mM NaCl, 5 mM KCl, 0,5 mM EGTA, 1 mM MgCl₂, pH 7,4) et centrifugés à 10000g pendant 20 minutes. Cette étape est répétée 2 fois, et le culot final, correspondant à la fraction mitochondriale, est conservé à -80 °C dans le tampon Tris. Le contenu protéique de la préparation est déterminé par la méthode de Lowry.

### b) Liaison du [³H]Ro 19-6327

Dans un tube Eppendorf, 100 µl de la préparation mitochondriale (2 mg protéine/ml) sont incubés pendant 1 heure à 37 °C en présence de 100 µl de [³H] Ro 19-6327 (33 nM, concentration finale) et 100 µl de tampon Tris contenant ou non les inhibiteurs. La réaction est arrêtée par l'addition de 1 ml de tampon Tris froid dans chaque tube, puis les échantillons sont centrifugés 2 minutes à 12000 g. Les surnageants sont aspirés et les culots lavés avec 1 ml de tampon Tris. Les culots sont ensuite solubilisés dans 200 µl de sodium dodécyl sulfate (20% poids/volume) pendant 2 heures à 70 °C. La radioactivité est déterminée par comptage des échantillons en scintillation liquide.

### c) Résultats

Le composé de l'exemple 1 décrit ci-dessus présente une CI₅₀ inférieure à 25 µM.

## Revendications

1. Utilisation d'un produit de formule générale **(I)** 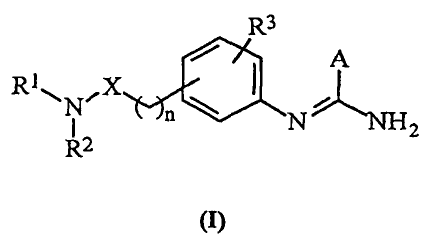
dans laquelle :
R¹ et R² représentent, indépendamment, un atome d'hydrogène ou un radical alkyle cycloalkyle, alkényle, alkynyle, allènyle, allènylalkyle, cyanoalkyle, -(CH₂)g-Z¹R⁴ ou -(CH₂)ₖ-COR⁵,
Z¹ représentant -O-, -NR⁶- , -S- ou une liaison,
R⁴ et R⁶ représentant, indépendamment, un atome d'hydrogène ou un radical alkyle alkényle, allènylalkyle, alkynyle, alkoxy ou cyanoalkyle,
R⁵ représentant un radical alkyle, allènyle, allènylalkyle, alkényle, alkynyle; cyanoalkyle, alkoxy ou NR⁷R⁸,
R⁷ et R⁸ représentant, indépendamment, un atome d'hydrogène ou un radical alkyle, allènyle, allènylalkyle, alkényle, alkynyle, cyanoalkyle ou alkoxy,
ou R¹ et R² pris ensemble forment avec l'atome d'azote un hétérocycle non aromatique de 4 à 8 chaînons, les éléments de la chaîne étant choisis parmi un groupe composé de -CH(R⁹)-, -NR¹⁰-, -O-, -S-, -CO-, ledit hétérocycle pouvant être substitué par un ou plusieurs substituants -(CH₂)ₖ-Z²R¹¹ ou -(CH₂)ₖ-COR¹²,
Z² représentant -O-, -NR¹³- ou -S- ou une liaison,
R¹¹, à chaque fois qu'il intervient, représentant indépendamment un atome d'hydrogène, un radical alkyle, alkényle, alkynyle, alkoxy, allènyle, allènylalkyle ou cyanoalkyle,
R¹³, à chaque fois qu'il intervient, représentant, indépendamment, un atome d'hydrogène ou un radical alkyle, alkényle, alkynyle, alkoxy, allènyle, allènylalkyle ou cyanoalkyle,
R¹², à chaque fois qu'il intervient, représentant un radical alkyle, allènyle, allènylalkyle, alkényle, alkynyle, cyanoalkyle, ou alkoxy ou NR¹⁴R¹⁵,
R¹⁴ et R¹⁵, à chaque fois qu'ils interviennent, représentant, indépendamment, un atome d'hydrogène ou un radical alkyle, alkoxy, allènyle, allènylalkyle, alkényle, alkynyle ou cyanoalkyle,
R⁹ et R¹⁰, à chaque fois qu'ils interviennent, représentant indépendamment un atome d'hydrogène, -(CH₂)ₖ-Z³R¹⁶ ou -(CH₂)ₖCOR¹⁷,
Z³ représentant -O-, -NR¹⁸-, -S- ou une liaison
R¹⁸ représentant, inépendamment, un atome d'hydrogène ou un radical alkyle, alkényle allènyle, allènylalkyle, alkynyle, alkoxy ou cyanoalkyle,
R¹⁶, à chaque fois qu'il intervient, représentant indépendamment un atome d'hydrogène ou un radical alkyle, alkényle, alkynyle, alkoxy, allènyle, allènylalkyle ou cyanoalkyle,
R¹⁷, à chaque fois qu'il intervient, représentant indépendamment un radical alkyle allènyle, allènylalkyle, alkényle, alkynyle, cyanoalkyle, alkoxy ou NR¹⁹R²⁰,
R¹⁹ et R²⁰ représentant, indépendamment, à chaque fois qu'il interviennent, un atome d'hydrogène ou un radical alkyle alkoxy, allènyle, allènylalkyle, alkényle, alkynyle ou cyanoalkyle ;
X représente un radical -CO- ou -(CH₂)ₘ- ;
R³ représente un atome d'hydrogène ou un radical alkyle ou alkoxy linéaire ou ramifié ayant de 1 à 6 atomes de carbone ;
A représente un radical alkyle linéaire ou ramifié ayant de 1 à 6 atomes de carbone ou un radical aryle carbocyclique ou hétérocyclique à 5 ou 6 chaînons contenant de 1 à 4 hétéroatomes choisis parmi O, S et N, ledit radical aryle étant éventuellement substitué par un ou plusieurs groupes choisis parmi les radicaux alkyle, alkényle ou alkoxy linéaires ou ramifiés ayant de 1 à 6 atomes de carbone ;
g, à chaque fois qu'il intervient, représentant, indépendamment, un entier de 1 à 6,
m, k et n, à chaque fois qu'ils interviennent, représentant, indépendamment, des entiers de 0 à 6 ;
étant entendu toutefois que, lorsque R³ représente un atome d'hydrogène ou un radical alkyle linéaire ou ramifié ayant de 1 à 6 atomes de carbone, alors R¹ et R² ne représentent pas, indépendamment, un atome d'hydrogène ou un radical alkyle, et NR¹R² ne représente en outre pas l'un des groupes pipéridinyle, morpholinyle, pyrrolidinyle non substitués ou le groupe pipérazinyle optionnellement substitué en position 4 par un radical alkyle comptant de 1 à 6 atomes de carbone ;
ou d'un sel pharmaceutiquement acceptable d'un tel composé ;
pour préparer un médicament destiné à traiter la schizophrénie, les dépressions, le psychoses, les troubles de la mémoire et de l'humeur, les troubles du comportement, 1; boulimie ou l'anorexie.

2. Utilisation selon la revendication 1, **caractérisé en ce que** le composé de formule générale **(I)** est tel que :
R¹ représente un radical alkyle et R² représentant l'un des radicaux alkyle, cycloalkyle alkényle, alkynyle, allènyle, cyanoalkyle, -(CH₂)_{g}-Z¹R⁴ ou -(CH₂)ₖ-COR⁵,
Z¹ représentant -O-, -NR⁶-, -S- ou une liaison,
R⁴ et R⁶ représentant un atome d'hydrogène ou un radical alkyle, alkényle, alkynyle alkoxy ou cyanoalkyle,
R⁵ représentant un radical alkyle, allènyle, allènylalkyle, alkényle, alkynyle cyanoalkyle, alkoxy ou NR⁷R⁸,
R⁷ et R⁸ représentant, indépendamment, allènyle, allènylalkyle, alkényle, alkynyle cyanoalkyle ou alkoxy,
ou R¹ et R² pris ensemble forment avec l'atome d'azote un hétérocycle non aromatique de 4 à 8 chaînons, les éléments de la chaîne étant choisis parmi un groupe composé de -CH(R⁹)-, -NR¹⁰- et -O-,
R⁹ et R¹⁰ représentant indépendamment un atome d'hydrogène ou un radical -(CH₂)ₖ₋Z³R¹⁶ 6 ou -(CH₂)ₖCOR¹⁷,
Z³ représentant une liaison,
R¹⁶ représentant indépendamment un atome d'hydrogène, un radical alkyle, alkényle, alkynyle, allènyle, allènylalkyle ou cyanoalkyle,
R¹⁷ représentant indépendamment un radical alkyle, allènyle, allènylalkyle, alkényle, alkynyle ou cyanoalkyle ;
n représente 0 ou 1 ;
k représente un entier de 1 à 6 ; et
X représente -(CH₂)ₘ₋ avec m représentant 0 ou 1.

3. Utilisation selon la revendication 1, **caractérisé en ce que** le composé de formule générale **(I)** est tel que :
R¹ représente un radical alkyle et R² représente l'un des radicaux alkyle, alkényle, alkynyle, allènyle ou cyanoalkyle,
ou R¹ et R² pris ensemble forment avec l'atome d'azote un hétérocycle non aromatique de 4 à 8 chaînons, les éléments de la chaîne étant choisis parmi un groupe composé de -CH(R⁹)-, -NR¹⁰- et -O-,
R⁹ et R¹⁰ représentant indépendamment un atome d'hydrogène ou un radical -(CH₂)ₖ-Z³R¹⁶,
Z³ représentant une liaison,
R¹⁶ représentant indépendamment un atome d'hydrogène ou un radical alkyle, alkynyle ou cyanoalkyle ;
n représente 0 ou 1 ;
k représente un entier de 1 à 3 ; et
X représente -(CH₂)ₘ- avec m représentant 0 ou 1.

4. Utilisation selon la revendication 1, **caractérisé en ce que** le composé de formule générale **(I)** est l'un des composés suivants :
- N'-(4-{[méthyl-(2-propynyl)amino]méthyl}phényl)-2-thiophènecarboximidamide ;
- N'-(4-{[méthyl(cyanométhyl)amino]méthyl}phényl)-2-thiophènecarboximidamide ;
- N'-(4-{[méthyl(propyl)amino]méthyl}phényl)-2-thiophènecarboximidamide ;
- N'-(4-{[méthyl(3-cyanoéthyl)amino]méthyl}phényl)-2-thiophènecarboximidamide ;
- N'-(4-{[méthyl(4-pentynyl)amino]méthyl}phényl)-2-thiophènecarboximidamide ;
ou un sel d'un de ces derniers.

5. Produit de formule générale **(I)** telle que définie dans la revendication 1 et dans laquelle :
R¹ représente un radical alkyle et R² représente l'un des radicaux alkényle, alkynyle, allènyle ou cyanoalkyle,
n représente 0 ou 1 ;
k représente un entier de 1 à 3 ; et
X représente -(CH₂)ₘ- avec m représentant 0 ou 1 ;
ou sel d'un tel composé.

6. Produit **caractérisé en ce qu'**il s'agit de l'un des composés suivants :
- N'-(4-{[méthyl-(2-propynyl)amino]méthyl}phényl)-2-thiophènecarboximidamide ;
- N'-(4-{[méthyl(cyanométhyl)amino]méthyl}phényl)-2-thiophènecarboximidamide ;
- N'-(4-{[méthyl(propyl)amino]méthyl}phényl)-2-thiophènecarboximidamide ;
- N'-(4-{[méthyl(3-cyanoéthyl)amino]méthyl}phényl)-2-thiophènecarboximidamide ;
- N'-(4-{[méthyl(4-pentynyl)amino]méthyl}phényl)-2-thiophènecarboximidamide ;
ou d'un sel d'un de ces derniers.

7. A titre de médicament, un composé de formule générale **(I)** telle que définie dans la revendication 1 et dans laquelle :
R¹ représente un radical alkyle et R² représente l'un des radicaux alkényle, alkynyle allènyle ou cyanoalkyle,
n représente 0 ou 1 ;
k représente un entier de 1 à 3 ; et
X représente -(CH₂)ₘ- avec m représentant 0 ou 1 ;
ou un sel pharmaceutiquement acceptable d'un de ces composés.

8. Composition pharmaceutique contenant, à titre de principe actif, un produit selon la revendication 5 ou un sel pharmaceutiquement acceptable d'un tel composé.

9. Utilisation d'un produit selon la revendication 5, ou d'un sel pharmaceutiquement acceptable dudit produit, pour préparer un médicament destiné à inhiber à la fois les NO synthases et les monoamine oxydases.

10. Utilisation d'un produit selon la revendication 5, ou d'un sel pharmaceutiquement acceptable dudit produit, pour préparer un médicament destiné à traiter la maladie de Parkinson, les démences séniles, la maladie d'Alzheimer, la chorée de Huntington, la sclérose latérale amyotrophique, la schizophrénie, les dépressions, les psychoses.

## Claims

1. Use of a product of general formula **(I)** 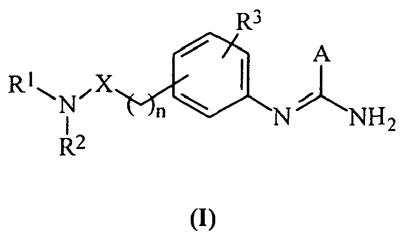
in which:
R¹ and R² represent, independently, a hydrogen atom or an alkyl, cycloalkyl, alkenyl, alkynyl, allenyl, allenylalkyl, cyanoalkyl, -(CH₂)_{g}-Z¹R⁴ or -(CH₂)ₖ-COR⁵ radical,
Z¹ representing -O-, -NR⁶- , -S- or a bond,
R⁴ and R⁶ representing, independently, a hydrogen atom or an alkyl, alkenyl, allenylalkyl, alkynyl, alkoxy or cyanoalkyl radical,
R⁵ representing an alkyl, allenyl, allenylalkyl, alkenyl, alkynyl, cyanoalkyl, alkoxy or NR⁷R⁸ radical,
R⁷ and R⁸ representing, independently, a hydrogen atom or an alkyl, allenyl, allenylalkyl, alkenyl, alkynyl, cyanoalkyl or alkoxy radical,
or R¹ and R² together with the nitrogen atom form a non-aromatic heterocycle with 4 to 8 members, the elements of the chain being chosen from a group comprising -CH(R⁹)-, -NR¹⁰-, -O-, -S-, -CO-, said heterocycle being able to be substituted by one or more substituents -(CH₂)ₖ-Z²R¹¹ or -(CH₂)ₖ-COR¹²,
Z² representing -O-, -NR¹³- or -S- or a bond,
R¹¹, each time that it occurs, representing independently a hydrogen atom, an alkyl, alkenyl, alkynyl, alkoxy, allenyl, allenylalkyl or cyanoalkyl radical,
R¹³, each time that it occurs, representing, independently, a hydrogen atom or an alkyl, alkenyl, alkynyl, alkoxy, allenyl, allenylalkyl or cyanoalkyl radical,
R¹², each time that it occurs, representing an alkyl, allenyl, allenylalkyl, alkenyl, alkynyl, cyanoalkyl, or alkoxy or NR¹⁴R¹⁵ radical,
R¹⁴ and R¹⁵, each time that they occur, representing, independently, a hydrogen atom or an alkyl, alkoxy, allenyl, allenylalkyl, alkenyl, alkynyl or cyanoalkyl radical,
R⁹ and R¹⁰, each time that they occur, representing independently a hydrogen atom, -(CH₂)ₖ-Z³R¹⁶ or -(CH₂)ₖCOR¹⁷,
Z³ representing -O-, -NR¹⁸-, -S- or a bond,
R¹⁸ representing, independently, a hydrogen atom or an alkyl, alkenyl, allenyl, allenylalkyl, alkynyl, alkoxy or cyanoalkyl radical,
R¹⁶, each time that it occurs, representing independently a hydrogen atom or an alkyl, alkenyl, alkynyl, alkoxy, allenyl, allenylalkyl or cyanoalkyl radical,
R¹⁷, each time that it occurs, representing independently an alkyl, allenyl, allenylalkyl, alkenyl, alkynyl, cyanoalkyl, alkoxy or NR¹⁹R²⁰ radical,
R¹⁹ and R²⁰ representing, independently, each time that they occur, a hydrogen atom or an alkyl alkoxy, allenyl, allenylalkyl, alkenyl, alkynyl or cyanoalkyl radical;
X represents a -CO- or -(CH₂)ₘ- radical;
R³ represents a hydrogen atom or a linear or branched alkyl or alkoxy radical having 1 to 6 carbon atoms;
A represents a linear or branched alkyl radical having 1 to 6 carbon atoms or a carbocyclic or heterocyclic aryl radical with 5 or 6 members containing 1 to 4 heteroatoms chosen from O, S, N, said aryl radical being optionally substituted by one or more groups chosen from the linear or branched alkyl, alkenyl or alkoxy radicals having 1 to 6 carbon atoms,
g, each time that it occurs, representing, independently, an integer from 1 to 6,
m, k and n, each time that they occur, representing, independently, integers from 0 to 6;
it being understood however that, when R³ represents a hydrogen atom or a linear or branched alkyl radical having 1 to 6 carbon atoms, then R¹ and R² do not represent, independently, a hydrogen atom or an alkyl radical, and moreover NR¹R² do not represent one of the non substituted piperidinyl, morpholinyl, pyrrolidinyl groups or the piperazinyl group optionally substituted in position 4 by an alkyl radical containing 1 to 6 carbon atoms;
or a pharmaceutically acceptable salt of such a compound;
for preparing a medicament intended to treat schizophrenia, depressions, psychoses, memory and mood disorders; behavioural disorders, boulimia or anorexia

2. Use according to claim 1, **characterized in that** the compound of general formula (I) is such that:
R¹ represents an alkyl radical and R² representing one of the alkyl, cycloalkyl, alkenyl, alkynyl, allenyl, allenylalkyl, cyanoalkyl, -(CH₂)_{g}-Z¹R⁴ or -(CH₂)ₖ-COR⁵ radicals;
Z¹ representing -O-, -NR⁶-, -S- or a bond,
R⁴ and R⁶ representing a hydrogen atom or an alkyl, alkenyl, alkynyl, alkoxy or cyanoalkyl radical,
R⁵ representing an alkyl, allenyl, allenylalkyl, alkenyl, alkynyl, cyanoalkyl, alkoxy or NR⁷R⁸ radical,
R⁷ and R⁸ representing, independently, allenyl, allenylalkyl, alkenyl, alkynyl, cyanoalkyl or alkoxy,
or R¹ and R² together with the nitrogen atom form a non aromatic heterocycle with 4 to 8 members, the elements of the chain being chosen from a group comprising -CH(R⁹)-, -NR¹⁰- and -O-,
R⁹ and R¹⁰ representing independently a hydrogen atom or a -(CH₂)ₖ-Z³R¹⁶ or -(CH₂)ₖCOR¹⁷ radical,
Z³ representing a bond,
R¹⁶ representing independently a hydrogen atom, an alkyl, alkenyl, alkynyl, allenyl, allenylalkyl or cyanoalkyl radical,
R¹⁷ representing independently an alkyl, allenyl, allenylalkyl, alkenyl, alkynyl or cyanoalkyl radical;
n represents 0 or 1;
k represents an integer from 1 to 6; and
X represents -(CH₂)ₘ- with m representing 0 or 1.

3. Use according to claim 1, **characterized in that** the compound of general formula **(I)** is such that:
R¹ represents an alkyl radical and R² represents one of the alkyl, alkenyl, alkynyl, allenyl or cyanoalkyl radicals,
or R¹ and R² together with the nitrogen atom form a non aromatic heterocycle with 4 to 8 members, the elements of the chain being chosen from a group comprising -CH(R⁹)-, -NR¹⁰- and -O-,
R⁹ and R¹⁰ representing independently a hydrogen atom or a -(CH₂)ₖ-Z³R¹⁶ radical, Z³ representing a bond,
R¹⁶ representing independently a hydrogen atom or an alkyl, alkynyl or cyanoalkyl radical;
n represents 0 or 1;
k represents an integer from 1 to 3; and
X represents -(CH₂)ₘ- with m representing 0 or 1.

4. Use according to claim 1, **characterized in that** the compound of general formula **(I)** is one of the following compounds:
- N'-(4-{[methyl-(2-propynyl)amino]methyl}phenyl)-2-thiophenecarboximidamide;
- N'-(4-{[methyl(cyanomethyl)amino]methyl}phenyl)-2-thiophenecarboximidamide;
- N'-(4-{[methyl(propyl)amino]methyl}phenyl)-2-thiophenecarboximidamide;
- N'-(4-{[methyl(3-cyanoethyl)amino]methyl}phenyl)-2-thiophenecarboximidamide;
- N'-(4-{[methyl(4-pentynyl)amino]methyl}phenyl)-2-thiophenecarboximidamide;
or a salt of one of the latter.

5. Product of general formula **(I)** as defined in claim 1 and in which
R¹ represents an alkyl radical and R² represents one of the alkenyl, alkynyl, allenyl or cyanoalkyl radical,
n represents 0 or 1 ;
k represents an integer from 1 to 3 ; and
X represents -(CH₂)ₘ- with m representing 0 or 1 ;
or a salt of such a compound.

6. Product **characterized in that** it is one of the following compounds:
- N'-(4-{[methyl-(2-propynyl)amino]methyl}phenyl)-2-thiophenecarboximidamide;
- N'-(4-([methyl(cyanomethyl)amino]methyl}phenyl)-2-thiophenecarboximidamide;
- N'-(4-[methyl(propyl)amino]methyl}phenyl)-2-thiophenecarboximidamide;
- N'-(4-{[methyl(3-cyanoethyl)amino]methyl}phenyl)-2-thiophenecarboximidamide;
- N'-(4-{[methyl(4-pentynyl)amino]methyl}phenyl)-2-thiophenecarboximidamide;
or a salt of one of the latter.

7. As a medicament, a compound of general formula **(I)** as defined in claim 1 and in which:
R¹ represents an alkyl radical and R² represents one of the alkenyl, alkynyl, allenyl or cyanoalkyl radicals,
n represents 0 or 1 ;
k represents an integer from 1 to 3 ; and
X represents -(CH₂)ₘ- with m representing 0 or 1 ;
or a pharmaceutically acceptable salt of one of these compounds.

8. Pharmaceutical composition containing, as active ingredient, a product according to claim 5 or a pharmaceutically acceptable salt of such a compound

9. Use of a product according to claim 5, or a pharmaceutically acceptable salt of said product, for preparing a medicament intended to inhibit both NO syntheses and monoamine oxydases.

10. Use of a product according to claim 5, or a pharmaceutically acceptable salt of said product, for preparing a medicament intended to treat Parkinson's disease, senile dementia, Alzheimer's disease, Huntington's chorea, amyotrophic lateral sclerosis, schizophrenia, depressions, psychoses.

## Patentansprüche

1. Verwendung eines Produkts der allgemeinen Formel (I) 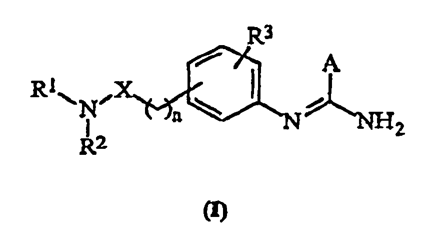
in der:
R¹ und R² unabhängig voneinander ein Wasserstofatom oder einen der Reste Alkyl, Cycloalkyl, Alkenyl, Alkinyl, Allenyl, Allenylalkyl, Cyanoalkyl, -(CH₂)_{g}-Z¹R⁴ oder (CH₂)ₖ-COR⁵ darstellen,
wobei Z¹ -O-, -NR⁶-, -S- oder eine Bindung darstellt,
R⁴ und R⁶ unabhängig voneinander ein Wasserstoffatom oder einen der Reste Alkyl, Alkenyl, Allenylalkyl, Alkinyl, Alkoxy oder Cyanoalkyl darstellen,
R⁵ einen der Reste Alkyl, Allenyl, Allenylalkyl, Alkenyl, Alkinyl, cyanoalkyl, Alkoxy oder NR⁷R⁸ darstellt,
R⁷ und R⁸ unabhängig voneinander ein Wasserstoffatom oder einen der Reste Alkyl, Allenyl, Allenylalkyl, Alkenyl, Alkinyl, Cyanoalkyl oder Alkoxy darstellen,
oder R¹ und R² zusammen mit dem Stickstoffatom einen nicht aromatischen Heterocyclus mit 4 bis 8 Gliedern darstellen, wobei die Elemente der Kette aus einer Gruppe ausgewählt sind, die aus -CH(R⁹)-, -NR¹⁰-, -O-, -S-, -CO- besteht, wobei dieser Heterocyclus mit einem oder mehreren Substituenten - (CH₂)ₖ-Z²R¹¹ oder - (CH₂)ₖ-COR¹² substituiert sein kann,
wobei _{Z}² -O-, -NR¹³- oder -S- oder eine Bindung darstellt,
R¹¹ jedes Mal unabhängig voneinander ein Wasserstoffatom oder einen der Reste Alkyl, Alkenyl, Alkinyl, Alkoxy, Allenyl, Allenylalkyl oder Cyanoalkyl darstellt,
R¹³ jedes Mal unabhängig voneinander ein Wasserstoffatom oder einen der Reste Alkyl, Alkenyl, Alkinyl, Alkoxy, Allenyl, Allenylalkyl oder cyanoalkyl darstellt,
R¹² jedes Mal einen der Reste Alkyl, Allenyl, Allenylalkyl, Alkenyl, Alkinyl, Cyanoalkyl oder Alkoxy oder NR¹⁴R¹⁵ darstellt,
R¹⁴ und R¹⁵ jedes Mal unabhängig voneinander ein Wasserstoffatom oder einen der Reste Alkyl, Alkoxy, Allenyl, Allenylalkyl, Alkenyl, Alkinyl oder Cyanoalkyl darstellen,
wobei R⁹ und R¹⁰ jedes Mal unabhängig voneinander ein Wasserstoffatom, - (CH₂)ₖ-Z³R¹⁶ oder - (CH₂)ₖCOR¹⁷ darstellen, Z³ -O-, -NR¹⁸-, -S- oder eine Bindung darstellt,
R¹⁸ unabhängig voneinander ein Wasserstoffatom oder einen der Reste Alkyl, Alkenyl, Allenyl, Allenylalkyl, Alkinyl, Alkoxy oder Cyanoalkyl darstellt,
R¹⁶ jedes Mal unabhängig voneinander ein Wasserstoffatom oder einen der Reste Alkyl, Alkenyl, Alkinyl, Alkoxy, Allenyl, Allenylalkyl oder Cyanoalkyl darstellt,
R¹⁷ jedes Mal unabhängig voneinander einen der Reste Alkyl, Allenyl, Allenylalkyl, Alkenyl, Alkinyl, Cyanoalkyl, Alkoxy oder NR¹⁹R²⁰ darstellt,
R¹⁹ und R²⁰ jedes Mal unabhängig voneinander ein Wasserstoffatom oder einen der Reste Alkyl, Alkoxy, Allenyl, Allenylalkyl, Alkenyl, Alkinyl oder Cyanoalkyl darstellen;
X einen Rest -CO- oder -(CH₂)ₘ- darstellt;
R³ ein Wasserstoffatom oder einen linearen oder verzweigten Alkyl- oder Alkoxyrest mit 1 bis 6 Kohlenstoffatomen darstellt;
A einen linearen oder verzweigten Alkylrest mit 1 bis 6 Kohlenstoffatomen oder einen carbocyclischen oder heterocyclischen Arylrest mit 5 bis 6 Gliedern darstellt, der 1 bis 4 Heteroatome enthält, die aus O, S und N ausgewählt sind, wobei der Arylrest ggf. mit einem oder mehreren Gruppen substituiert ist, die aus den linearen oder verzweigten Resten Alkyl, Alkenyl oder Alkoxy mit 1 bis 6 Kohlenstoffatomen ausgewählt sind;
g jedes Mal unabhängig voneinander eine ganze Zahl von 0 bis 6 darstellt, m, k und n jedes Mal unabhängig voneinander ganze Zahlen von 0 bis 6 darstellen;
wobei jedoch gilt, dass, wenn R³ ein Wasserstoffatom oder einen linearen oder verzweigten Alkylrest mit 1 bis 6 Kohlenstoffatomen darstellt, dann R¹ und R² unabhängig voneinander nicht ein Wasserstoffatom oder einen Alkylrest darstellen und NR¹R² außerdem nicht eine der nicht substituierten Gruppen Piperidinyl, Morpholinyl, Pyrrolidinyl oder die Piperazinylgruppe darstellt, die gegebenenfalls in Position 4 mit einem Alkylrest mit 1 bis 6 Kohlenstoffatomen substituiert ist;
oder eines pharmazeutisch annehmbaren Salzes einer solchen Verbindung;
zur Herstellung eines Medikaments für die Behandlung von Schizophrenie, Depressionen, Psychosen, Gedächtnis- und Stimmungsstörungen, Verhaltensstörungen, Bulimie oder Anorexie.

2. Verwendung nach Anspruch 1, **dadurch gekennzeichnet, dass** die Verbindung der allgemeinen Formel (I) so beschaffen ist, dass:
R¹ einen Alkylrest darstellt und R² einen der Reste Alkyl, cycloalkyl, Alkenyl, Alkinyl, Allenyl, Cyanoalkyl, - (CH₂)_{g}-Z¹R⁴ oder - (CH₂)ₖ-COR⁵ darstellt,
wobei Z¹ -O-, -NR⁶-, -S- oder eine Bindung darstellt,
R⁴ und R⁶ ein Wasserstoffatom oder einen der Reste Alkyl, Alkenyl, Alkinyl, Alkoxy oder Cyanoalkyl darstellen,
R⁵ einen der Reste Alkyl, Allenyl, Allenylalkyl, Alkenyl, Alkinyl, Cyanoalkyl, Alkoxy oder NR⁷R⁸ darstellt,
R⁷ und R⁸ unabhängig voneinander Allenyl, Allenylalkyl, Alkenyl, Alkinyl, Cyanoalkyl oder Alkoxy darstellen,
oder R¹ und R² zusammen mit dem Stickstoffatom einen nicht aromatischen Heterocyclus mit 4 bis 8 Gliedern bilden, wobei die Elemente der Kette aus einer Gruppe ausgewählt sind, die aus -CH(R⁹)-, -NR¹⁰- oder -O- besteht,
R⁹ und R¹⁰ unabhängig voneinander ein wasserstoffatom oder einen Rest - (CH₂)ₖ-Z³R¹⁶ oder - (CH₂)ₖCOR¹⁷ darstellen,
Z³ eine Bindung darstellt,
R¹⁶ unabhängig voneinander ein Wasserstoffatom oder einen der Reste Alkyl, Alkenyl, Alkinyl, Allenyl, Allenylalkyl oder Cyanoalkyl darstellt,
R¹⁷ unabhängig voneinander einen der Reste Alkyl, Allenyl, Allenylalkyl, Alkenyl, Alkinyl oder Cyanoalkyl darstellt;
n 0 oder 1 darstellt;
k eine ganze Zahl von 1 bis 6 darstellt; und
X -(CH₂)ₘ- darstellt, wobei m 0 oder 1 darstellt.

3. Verwendung nach Anspruch 1, **dadurch gekennzeichnet, dass** die Verbindung der allgemeinen Formel (I) so beschaffen ist, dass:
R¹ einen Alkylrest darstellt und R² einen der Reste Alkyl, Alkenyl, Alkinyl, Allenyl oder Cyanoalkyl darstellt,
oder R¹ und R² zusammen mit dem Stickstoffatom einen nicht aromatischen Heterocyclus mit 4 bis 8 Gliedern bilden, wobei die Elemente der Kette aus einer Gruppe ausgewählt sind, die aus -CH(R⁹)-, -NR¹⁰- oder -O- besteht,
wobei R⁹ und R¹⁰ unabhängig voneinander ein Wasserstoffatom oder einen Rest -(CH₂)ₖ-Z³R¹⁶ darstellen,
Z³ eine Bindung darstellt,
R¹⁶ unabhängig voneinander ein Wasserstoffatom oder einen Alkyl-, Alkinyl- oder Cyanoalkylrest darstellt;
n 0 oder 1 darstellt;
k eine ganze Zahl von 1 bis 3 darstellt; und
X - (CH₂)ₘ- darstellt, wobei m 0 oder 1 darstellt.

4. Verwendung nach Anspruch 1, **dadurch gekennzeichnet, dass** die Verbindung der allgemeinen Formel (I) eine der folgenden Verbindungen ist:
- N'-(4-{[Methyl(2-propinyl)amino]methyl}phenyl)-2-thiophencarboximidamid;
- N'-(4-{[Methyl(cyanomethyl)amino]methyl}phenyl)-2-thiophencarboximidamid;
- N'-(4-{[Methyl(propyl)amino]methyl}phenyl)-2-thiophencarboximidamid;
- N'-(4-{[Methyl(3-Cyanoethyl)amino]methyl}phenyl)-2-thiophencarboximidamid;
- N'-(4-{[Methyl(4-pentinyl)amino]methyl}phenyl)-2-thiophencarboximidamid;
oder ein Salz einer dieser Verbindungen.

5. Produkt der allgemeinen Formel (I), wie sie in Anspruch 1 definiert ist, in der:
R¹ einen Alkylrest darstellt und R² einen der Reste Alkenyl, Alkinyl, Allenyl oder Cyanoalkyl darstellt,
n 0 oder 1 darstellt;
k eine ganze Zahl von 1 bis 3 darstellt; und
X - (CH₂)ₘ- darstellt, wobei m 0 oder 1 darstellt;
oder ein Salz einer solchen Verbindung.

6. Produkt, **dadurch gekennzeichnet, dass** es sich um eine der folgenden Verbindungen handelt:
- N'-(4-{[Methyl(2-propinyl)amino]methyl}phenyl)-2-thiophencarboximidamid;
- N'-(4-{[Methyl(cyanomethyl)aminol]methyl}phenyl)-2-thiophencarboximidamid;
- N'-(4-{[Methyl(propyl)amino]methyl}phenyl)-2-thiophencarboximidamid;
- N'-(4-{[Methyl(3-cyanoethyl)amino]methyl}phenyl)-2-thiophencarboximidamid;
- N'-(4-{[Methyl(4-pentinyl)amino]methyl}phenyl)-2-thiophencarboximidamid;
oder ein Salz einer dieser Verbindungen.

7. Eine Verbindung der allgemeinen Formel (I), wie sie in Anspruch 1 definiert ist, in der:
R¹ einen Alkylrest darstellt und R² einen der Reste Alkenyl, Alkinyl, Allenyl oder Cyanoalkyl darstellt,
n 0 oder 1 darstellt;
k eine ganze Zahl von 1 bis 3 darstellt; und
X -(CH₂)ₘ- darstellt, wobei m 0 oder 1 darstellt;
oder ein pharmazeutisch annehmbares Salz einer dieser Verbindungen in Form eines Medikaments.

8. Pharmazeutische Zusammensetzung, die als Wirkstoff ein Produkt nach Anspruch 5 oder ein pharmazeutisch annehmbares Salz einer solchen Verbindung enthält.

9. Verwendung eines Produkts nach Anspruch 5 oder eines pharmazeutisch annehmbaren Salzes dieses Produkts für die Herstellung eines Medikaments, das für die Hemmung der NO-Synthasen und gleichzeitig der Monoaminooxidasen bestimmt ist.

10. Verwendung eines Produkts nach Anspruch 5 oder eines pharmazeutisch annehmbaren Salzes dieses Produkts für die Herstellung eines Medikaments, das für die Behandlung der Parkinson-Krankheit, von Altersdemenzen, der Alzheimer-Krankheit, der Huntington-Chorea, der amyotrophen Lateralsklerose, der Schizophrenie, von Depressionen und Psychosen bestimmt ist.
